(19) Europäisches Patentamt
European Patent Office
Office européen des brevets

(11) Veröffentlichungsnummer: **0 156 105**
**B1**

(12) # EUROPÄISCHE PATENTSCHRIFT

(45) Veröffentlichungstag der Patentschrift: **01.03.89**

(51) Int. Cl.⁴: **A 61 K 6/06,** A 61 K 6/08

(21) Anmeldenummer: **85100351.7**

(22) Anmeldetag: **15.01.85**

(54) **Dentales Füllungsmaterial.**

(30) Priorität: **30.01.84 DE 3403040**

(43) Veröffentlichungstag der Anmeldung:
**02.10.85 Patentblatt 85/40**

(45) Bekanntmachung des Hinweises auf die Patenterteilung:
**01.03.89 Patentblatt 89/9**

(84) Benannte Vertragsstaaten:
**AT BE CH DE FR GB IT LI LU NL SE**

(56) Entgegenhaltungen:
**EP-A- 0 026 398**
**EP-A- 0 060 911**
**EP-A- 0 091 990**
**WO-A-81/01366**
**WO-A-81/02254**
**DE-A- 2 405 578**
**FR-A- 2 370 467**

**RESEARCH DISCLOSURE, Nr. 162, Oktober 1977, Seite 80, Nr. 16269, Havant, US; "New dental materials"**

**Die Akte enthält technische Angaben, die nach dem Eingang der Anmeldung eingereicht wurden und die nicht in dieser Patentschrift enthalten sind.**

(73) Patentinhaber: **Blendax GmbH, Rheinallee 88, D-6500 Mainz (DE)**

(72) Erfinder: **Kuhlmann, Werner, Dr., Vogelsbergstrasse 11, D-6500 Mainz-Hechtsheim (DE)**

EP 0 156 105 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues dentales Füllungsmaterial, das ein spezielles Füllstoffgemisch enthält.

Dentale Restorationsmaterialien auf der Basis polymerisierbarer Verbindungen, sogenannte «Composites», enthalten neben einem oder mehreren polymerisierbaren Monomeren, Aktivatoren, ggf. Polymerisationskatalysatoren und sonstigen Bestandteilen obligatorisch einen mineralischen Füllstoff.

Dieser Füllstoff ist nach Art und Menge bestimmend für die physikalischen Eigenschaften der durch das Composite hergestellten Füllung. Je höher der Füllstoffanteil und dessen Teilchengrößen, desto besser für die physikalischen Eigenschaften, desto schlechter jedoch in der Regel die Polierbarkeit.

Man hat deshalb versucht, die Polierbarkeit derartiger Materialien durch den Einsatz von Füllstoffen mit niederen Teilchengrößen zwischen etwa 10 und 300 Nanometern zu verbessern; dies geht jedoch auf Kosten der mechanischen Eigenschaften.

Diese sogenannten «Mikrofiller» finden insbesondere überwiegend oder nahezu ausschließlich Verwendung bei der Herstellung von sogenannten lichthärtenden Composites, d.h. dentalen Restorationsmaterialien, die in einer Phase vorliegen und Füllstoffe, polymerisierbare Verbindungen und einen unter Lichteinfluß Radikale bildenden Polymerisationsinitiator enthalten.

Dies beruht insbesondere darauf, daß diese Materialien eine bestimmte Aushärtungstiefe aufweisen müssen, die mit den meisten Füllstoffen höherer Teilchengrößen, den sogenannten «Makrofillern», nicht erreicht wird.

Andere Makrofiller, die diesen Nachteil nicht aufweisen, verursachen Verfärbungen bei der Polymerisation (= Aushärtung) der Füllung. Dies gilt insbesondere für die verschiedenen Glassorten, die bei alleiniger Benutzung eine grünliche oder gräuliche Verfärbung während der Aushärtung der Füllung ergeben und außerdem auch nicht polierbar sind.

Darüber hinaus ist auch eine Röntgenopazität von gehärteten Zahnfüllungen erwünscht, die mit Mikrofüllstoffen auf Basis von Siliciumdioxid nicht erreicht werden kann.

Es bestand daher ein Bedürfnis, dentale Füllungsmaterialien zu entwickeln, die diese Nachteile nicht aufweisen, erforderlichenfalls auch ohne Polymerisationskatalysatoren unter Lichteinfluß gut aushärtbar sind, keine Verfärbung ergeben, jedoch gute physikalische Eigenschaften aufweisen, insbesondere im Hinblick auf eine verringerte Wasserabsorption und Schrumpfung, einen auf Null hin tendierenden thermischen Ausdehnungskoeffizienten und verbesserte mechanische Eigenschaften, insbesondere hinsichtlich Härte und diametraler Zugfestigkeit, und röntgenopak sind.

Darüber hinaus ist es erwünscht, wenigstens eine befriedigende Polierbarkeit zu erreichen.

Es wurde nun gefunden, daß sich ein dentales Füllungsmaterial mit den beschriebenen Eigenschaften herstellen läßt, wenn man in einem solchen Mittel als anorganisches Füllungsmaterial in einer Menge von 60 bis 90 Gew.-% der Gesamtzusammensetzung ein Gemisch aus a) mindestens einem silanisierten röntgenopaken anorganischen Füllstoff mit einer Korngrößenverteilung zwischen 0,5 und 40 µm in einer Menge von 5 bis 20, insbesondere 10 bis 15 Gew.-% des Füllstoffgemisches, b) mindestens einem silanisierten röntgenopaken anorganischen Füllstoff mit einer Korngrößenverteilung zwischen 0,2 und 15 µm in einer Menge von 20 bis 35, insbesondere 25 bis 35 Gew.-% des Füllstoffgemisches, und c) einen silanisierten Siliciumdioxid-Mikrofüllstoff, mit einer Korngrößenverteilung zwischen 5 und 150 nm in einer Menge von 45 bis 75, insbesondere 55 bis 65 Gew.-% des Füllstoffgemisches verwendet.

Die präzise Korngrößenverteilung des in einer Menge von 5 bis 20, vorzugsweise 10 bis 15, insbesondere 10 Gew.-% des Gesamtfüllstoffs enthaltenen röntgenopaken Füllstoffs mit größerem mittleren Teilchendurchmesser ist in der Regel die folgende:

| | |
|---|---|
| ~5% | 0,5– 1 µm |
| ~10% | 1 – 2 µm |
| ~20% | 2 – 5 µm |
| ~20% | 5 –10 µm |
| ~30% | 10 –20 µm |
| ~15% | 20 –40 µm |

Die exakte Korngrößenverteiling des in einer Menge von 20 bis 35, vorzugsweise 25 bis 35, insbesondere 30 Gew.-% des Gesamtfüllstoffs enthaltenen röntgenopaken Füllstoffs mit kleinerem mittlerem Teilchendurchmesser geht aus der folgenden Tabelle hervor:

| | |
|---|---|
| ~5% | 0,2– 0,5 µm |
| ~10% | 0,5– 1 µm |
| ~15% | 1 – 2 µm |
| ~45% | 2 – 5 µm |
| ~20% | 5 –10 µm |
| ~5% | 10 –15 µm |

Die Korngröße des in einer Menge von 45 bis 75, vorzugsweise 55 bis 65, insbesondere 60 Gew.-%, berechnet auf den Gesamtfüllstoff, enthaltenen Mikrofüllstoffs liegt zwischen 5 bis 150, vorzugsweise 120 nm; die bevorzugte mittlere Teilchengröße beträgt 30 bis 60, vorzugsweise 40 nm.

Die röntgenopaken Komponenten a) und b) mit unterschiedlichen Teilchengrößen können chemisch gleich oder unterschiedlich zusammengesetzt sein.

Um die Einarbeitbarkeit des erfindungsgemäßen Füllstoffgemisches in die Zusammensetzung und die Verträglichkeit mit den organischen Bestandteilen zu verbessern, ist es notwendig, diese Füllstoffe mit einem Organosilan zu silanisieren. Die Silanisierung kann mit jedem geeigneten Organosilan der allgemeinen Formel

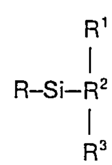

wobei R, $R^1$, $R^2$ und $R^3$ gleiche oder verschiedene organische Reste darstellen mit der Maßgabe, daß mindestens ein Rest eine OH-Gruppe oder einen in eine OH-Gruppe, beispielsweise durch Hydrolyse, überführbaren Rest, insbesondere eine Alkoxygruppe, bedeutet, erfolgen. Bevorzugte Organosilane sind (Meth)Acroylpropyldihydroxymethoxysilan, (Meth)Acroylpropylhydroxidimethoxysilan, (Meth)Acroylpropyltrimethoxysilan oder deren Gemische; jedoch sind beispielsweise auch Vinyltriethoxysilan oder Vinyltri(methoxyethoxy)silan geeignete Silanisierungsmittel.

Ein geeigneter Mikrofiller ist insbesondere gefälltes oder pyrogenes Siliciumdioxid, beispielsweise vom Typ «Aerosil®». Dieses ist vorzugsweise silanisiert.

Ein geeignetes Material ist in der DE-OS 2 403 211 sowie insbesondere in der EP-OS 60 911 beschrieben.

Wie bereits ausgeführt, eignen sich die erfindungsgemäßen dentalen Füllungsmaterialien insbesondere zur Anwendung als lichthärtende Produkte, d.h., Produkte, die in einer Phase vorliegen und unter Einwirkung von Licht polymerisieren.

Solche Zusammensetzungen enthalten einen oder mehrere Photopolymerisationsinitiatoren. Als solche sind insbesondere Carbonylverbindungen wie Benzoin und dessen Derivate, insbesondere Benzoinmethyläther, Benzil und Benzilderivate, beispielsweise 4,4-Oxidibenzil oder andere Dicarbonylverbindungen, z.B. Diacetyl, 2,3-Pentandion oder Metallcarbonyle, Chinone, insbesondere Campherochinon, oder deren Derivate geeignet. Der Anteil an Photopolymerisationsinitiator beträgt 0,01 bis 5 Gew.-% der Gesamtzusammensetzung.

Diese im Licht härtbaren, d.h. photopolymerisierbaren Präparate enthalten vorzugsweise auch noch sogenannte Polymerisationsbeschleuniger. Dies sind Substanzen, die in Gegenwart von Polymerisationsinitiatoren die Polymerisationsreaktion beschleunigen. Bekannte Beschleuniger sind beispielsweise Amine wie p-Toluidin, N,N-Dimethyl-p-toluidin, N,N-Di(hydroxyethyl)-p-toluidin, Trialkylamine wie Trehexylamin, Polyamine wie N,N,N',N'-Tetraalkylalkylendiamine, Barbitursäure und Dialkylbarbitursäuren und Sulfimide, vorzugsweise in einer Menge von 0,01 bis 5 Gew.-% der Gesamtzusammensetzung.

Geeignete Acceleratoren sind beispielsweise bei G.M. Brauer et al., Journal of Dental Research, Vol. 58/No. 10 (1979), S. 1994–2000, beschrieben.

Ein bevorzugtes lichthärtbares dentales Füllungsmaterial enthält 60 bis 90 Gew.-% der Gesamtzusammensetzung eines anorganischen Füllstoffgemisches der eingangs definierten Art.

Die bevorzugte Korngrößenverteilung des Gesamtfüllstoffgemisches läßt sich in der folgenden Tabelle darstellen:

| | | |
|---|---|---|
| 60% | 0,005–120 nm | (pyrogenes $SiO_2$, mittl. Primärteilchengrösse ~ 40 nm) |
| 1,5% | 0,2– 0,5 µm | |
| 3,5% | 0,5– 1 µm | |
| 5% | 1 – 2 µm | |
| 16% | 2 – 5 µm | |
| 8% | 5 –10 µm | |
| 5% | 10 –20 µm | |
| 1% | 20 –40 µm | |

Es ist selbstverständlich möglich, die erfindungsgemäßen deentalen Füllungsmaterialien auch als zweiphasige Präparate einzusetzen, von denen die eine Phase einen Polymerisationskatalysator, beispielsweise ein Peroxid, und die andere Phase einen Beschleuniger für dieses Peroxid, beispielsweise ein organisches Amin, enthält, wobei das Zusammenbringen beider Phasen unmittelbar vor der Zahnfüllung erfolgt und die Polymerisation in der aufgebohrten, vorzugsweise mit einem Unterfütterungs- oder einem Bondingmaterial versehenen, zu füllenden Kavität eintritt.

Geeignete Peroxide, die bei der Auslösung der Polymerisation unter Radikalbildung zerfallen, sind beispielsweise Arylperoxide wie Benzoylperoxid, Cumolhydroperoxid, Harnstoffperoxid, tert.-Butylhydroperoxid oder -perbenzoat und Silylperoxide, vorzugsweise in Mengen von 0,01 bis 5, insbesondere 0,5 bis 2,5 Gew.-% der Gesamtzusammensetzung.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin, oder Barbitursäure oder deren Derivate, beispielsweise eine Dialkylbarbitursäure, zugesetzt.

Als polymerisierbare Monomere in den erfindungsgemäßen dentalen Füllungsmaterialien sind prinzipiell alle für diesen Zweck vorgeschlagenen und geeigneten Verbindungen einsetzbar. Hier seien insbesondere die bekannten Reaktionsprodukte aus Bisphenolen, insbesondere Bisphenol A, und Glycidylmethacrylat, unter der Abkürzung Bis-GMA bekannt, die verschiedenen Alkandioldimethacrylate wie 1,6-Hexandiolmethacrylat, 1,4-Butandioldimethacrylat, Tri- oder Tetraethylenglykoldimethacrylat, Bis-(2-methacroylpropyl)-phthalat, -isophthalat oder -terephthalat, Trimethylolpropandi- und trimethacrylat, sowie insbesondere die Reaktionsprodukte aus Diisocyanaten und Hydroxyalkylmethacrylaten, wie sie beispielsweise in der DE-OS 2 312 559 beschrieben sind, Addukte aus (Di)Isocyanaten und 2,2-Propan-bis-[3-(4-phenoxy)-1,2-hydroxypropan]-1-methacrylat nach der US-PS 3 629 187 sowie insbesondere die Addukte aus Isocyanaten und Methacroylalkylethern, -alkoxybenzolen bzw. -alkoxycycloalkanen, wie sie in der EP-OS 44 352 beschrieben sind, genannt.

Selbstverständlich können auch Gemische aus geeigneten Monomeren verwendet werden.

Es ist schließlich zweckmäßig, dentalen Füllungsmaterialien auf Kunststoffbasis UV-Stabilisatoren zuzusetzen, und das Nachdunkeln während des Alterns der Füllungen zu vermeiden. Ein besonders geeigneter UV-Stabilisator ist 2-Hydroxy-4-methoxybenzophenon. Ein weiteres bevorzugtes Material ist 2-(2'-Hydroxy-5'-methylphenyl)-benzotriazol; jedoch ist prinzipiell jedes physiologisch inerte UV-absorbierende Agens für diesen Zweck geeignet.

So seien beispielhaft noch Hydrochinon, p-Benzochinon, P-Butylhydroxytoluol u.ä. genannt. Die letztere Verbindung kann beispielsweise auch als Antioxidans in der Füllung wirken.

Eine Übersicht über die in dentalen Füllungsmaterialien üblicherweise zum Einsatz gelangenden Substanzen findet sich in dem Artikel von R.L. Bowen im Journal of Dental Research, Vol. 58/5 (Mai 1979), S. 1493 bis 1503, sowie der daran angeschlossenen Ergänzung von J.F. Lann, S. 1504 bis 1506.

Zur Einstellung eines möglichst naturgetreuen Eindrucks der gefüllten Zahnflächen enthalten Composite-Materialien erforderlichenfalls auch einen geringen Anteil an Farbstoffen oder Pigmenten.

Die nachfolgenden Beispiele dienen der Erläuterung der Erfindung.

**Beispiel 1**

| | |
|---|---|
| Pyrogenes Siliciumdioxid vom Typ «Aerosil®» (mittlere Teilchengrösse 40 nm) | 42,0 (Gew.-Teile) |
| Bariumboraluminiumsilikatglas (Teilchengrösse 0,2–15 μm) | 21,0 |
| Bariumaluminiumsilikatglas (Teilchengrösse 0,5–40 μm) | 7,0 |
| Methacroylpropyltrihydroxysilan (auf die Füllstoffe aufgebracht) | 3,0 |
| Reaktionsprodukt aus Glycidylmethacrylat und Bisphenol A (Bis-GMA) | 15,5 |
| Triethylenglykoldimethacrylat | 10,5 |
| Campherochinon | 0,15 |
| Ethylbenzoin | 0,15 |
| Dimethylaminoethylmethacrylat | 0,4 |
| Hydrochinonmonomethylether, UV-Stabilisator, Farbstoffe | q.s. |

Nach dem Aushärten unter Lichteinfluß wurde eine röntgenopake, polierfähige Füllung erhalten.

Beispiel 2

| | Teil A | Teil B |
|---|---|---|
| Pyrogenes Siliciumdioxid (mittl. Teilchengrösse 50 nm) | 40 | 40 (Gew.-Teile) |
| Bariumaluminiumsilikatglas (Teilchengrösse 0,2–15 μm) | 22 | 22 |
| Bariumaluminiumsilikatglas (Teilchengrösse 0,5–40 μm) | 6,5 | 6,5 |
| Methacroylpropyltrihydroxysilan (aufgebracht auf die Füllstoffe) | 3,2 | 3,2 |
| Bis-GMA | 15,8 | 15,8 |
| 1,6-Hexandioldimethacrylat | 10,5 | 10,6 |
| Benzoylperoxid | – | 0,4 |
| N,N-Diethanolo-p-toluidin | 0,5 | – |
| UV-Absorber, Stabilisatoren, Farbstoffe | q.s. | q.s. |

Nach dem Zusammenbringen beider Teile wird ein polierfähiges, röntgenopakes Polymerisat mit ausgezeichneten physikalischen Eigenschaften erhalten.

Beispiel 3

| | |
|---|---|
| Kolloidale Kieselsäure (mittlere Teilchengrösse 60 nm, Organosilikon-Gehalt 8%) | 44,5 (Gew.-Teile) |
| Bariumaluminiumsilikatglas (Silanisiert; Teilchengrösse 0,2–15 μm) | 22,3 |
| Lanthanglas (Silanisiert; Teilchengrösse 1–30 μm) | 7,6 |

| | |
|---|---|
| Bis-GMA | 10,5 |
| 1,6-Hexandioldimethacrylat | 8,5 |
| Trimethylolpropantrimethacrylat | 5,8 |
| Diethylaminoethylmethacrylat | 0,4 |
| Benzil | 0,4 |
| UV-Absorber, Stabilisator, Farbstoffe | q.s. |

Nach der durch Lichteinwirkung erfolgten Aushärtung wurde ein farbstabiles, röntgenopakes, polierfähiges Material mit guten mechanischen Eigenschaften erhalten.

## Patentansprüche

1. Dentales Füllungsmaterial, enthaltend eine oder mehrere polymerisierbare (Meth)Acrylverbindungen, Polymerisationsbeschleuniger und/oder Polymerisationsinitiatoren und gegebenenfalls weitere in solchen Mitteln an sich übliche Zusätze sowie 60 bis 90 Gew.-%, berechnet auf die Gesamtzusammensetzung eines anorganischen Füllstoffgemisches, dadurch gekennzeichnet, daß es als Füllstoffgemisch ein Gemisch aus

a) 5 bis 20 Gew.-%, bezogen auf das Füllstoffgemisch, mindestens eines silanisierten röntgenopaken Füllstoffs mit einer Korngrößenverteilung zwischen 0,5 und 40 µm,

b) 20 bis 35 Gew.-%, bezogen auf das Füllstoffgemisch, mindestens eines silanisierten röntgenopaken Füllstoffs mit einer Korngrößenverteilung zwischen 0,2 und 15 µm, und

c) 45 bis 75 Gew.-%, bezogen auf das Füllstoffgemisch, eines silanisierten Siliciumdioxid-Mikrofüllstoffs mit einer Korngrößenverteilung zwischen 5 und 150 nm enthält.

2. Dentales Füllungsmaterial nach Anspruch 1, dadurch gekennzeichnet, daß es 10 bis 15 Gew.-%, berechnet auf das Füllstoffgemisch, des Füllstoffs a) enthält.

3. Dentales Füllungsmaterial nach Anspruch 1, dadurch gekennzeichnet, daß es 25 bis 35 Gew.-%, berechnet auf das Füllstoffgemisch, des Füllstoffs b) enthält.

4. Dentales Füllungsmaterial nach Anspruch 1, dadurch gekennzeichnet, daß es 55 bis 65 Gew.-%, bezogen auf das Füllstoffgemisch, eines Mikrofüllstoffs c) enthält.

5. Dentales Füllungsmaterial nach Anspruch 1 und/oder 4, dadurch gekennzeichnet, daß der Mikrofüllstoff c) eine durchschnittliche Teilchengröße von 30 bis 60 nm aufweist.

6. Dentales Füllungsmaterial nach einem oder mehreren der vorhergehenden Ansprüche, dadurch gekennzeichnet, daß der Füllstoff a) folgende Korngrößenverteilung aufweist:

| | |
|---|---|
| 5% | 0,5– 1 µm |
| 10% | 1 – 2 µm |
| 20% | 2 – 5 µm |
| 20% | 5 –10 µm |
| 30% | 10 –20 µm |
| 15% | 20 –40 µm |

7. Dentales Füllungsmaterial nach einem oder mehreren der vorhergehenden Ansprüche, dadurch gekennzeichnet, daß der Füllstoff b) folgende Korngrößenverteilung aufweist:

| | |
|---|---|
| 5% | 0,2– 0,5 µm |
| 10% | 0,5– 1 µm |
| 15% | 1 – 2 µm |
| 45% | 2 – 5 µm |
| 20% | 5 –10 µm |
| 5% | 10 –15 µm |

## Claims

1. Dental filling material containing one or more polymerizable (meth)acrylic compounds, polymerization accelerators and/or polymerization initiators and possibly further additives normally used in such agents, and 60 to 90% by weight, calculated to the total composition, of an inorganic filler mixture, characterized in that the filler mixture is composed of a mixture of

a) 5 to 20% by weight, related to the filler mixture of at least one silanized X-ray opaque filler with a particle size distribution between 0.5 and 40 µm,

b) 20 to 35% by weight, related to the filler mixture, of at least one silanized X-ray opaque filler with a particle size distribution between 0.2 and 15 µm, and

c) 45 to 75% by weight, related to the filler mixture, of a silanized silica microfiller with a particle size distribution between 5 and 150 nm.

2. Dental filler material according to claim 1, characterized in that it contains 10 to 15% by weight, calculated to the filler mixture, of filler a).

3. Dental filling material according to claim 1, characterized in that it contains 25 to 35% by weight, calculated to the filler mixture, of filler b).

4. Dental filling material according to claim 1, characterized in that it contains 55 to 65% by weight, calculated to the filler mixture, of microfiller c).

5. Dental filling material according to claim 1 and/or 4, characterized in that the microfiller c) has an average particle size of 30 to 60 nm.

6. Dental material according to one or more of the preceding claims, characterized in that the filler a) has the following particle size distribution:

| | |
|---|---|
| 5% | 0,5– 1 µm |
| 10% | 1 – 2 µm |
| 20% | 2 – 5 µm |
| 20% | 5 –10 µm |
| 30% | 10 –20 µm |
| 15% | 20 –40 µm |

7. Dental filling material according to one or more of the preceding claims, characterized in that the filler b) has the following particle size distribution:

| | |
|---|---|
| 5% | 0,2– 5 µm |
| 10% | 0,5– 1 µm |
| 15% | 1 – 2 µm |
| 45% | 2 – 5 µm |

| | |
|---|---|
| 20% | 5 –10 µm |
| 5% | 10 –15 µm |

## Revendications

1. Matière dentaire d'obturation, contenant un ou plusieurs composés (méth)acryliques polymérisables, des accélérateurs de polymérisation et/ou des initiateurs de polymérisation, et éventuellement d'autres additifs courants en soi dans de tels produits, ainsi que 60 à 90% en poids, calculés par rapport à la composition globale, d'un mélange de substances de remplissage inorganiques, caractérisée en ce que, comme mélange de substances de remplissage, celle contient un mélange

a) de 5 à 20% en poids, par rapport au mélange de substances de remplissage, d'au moins une substance de remplissage silanisée, opaque aux rayons X et présentant une répartition granulométrique entre 0,5 et 40 µm,

b) de 20 à 35% en poids, par rapport au mélange de substances de remplissage, d'au moins une substance de remplissage silanisée, opaque aux rayons X et présentant une répartition granulométrique entre 0,2 et 15 µm, et

c) de 45 à 75% en poids, par rapport au mélange de substances de remplissage, d'une micro-substance de remplissage à base de dioxyde de silicium, silanisée et présentant une répartition granulométrique entre 5 et 150 nm.

2. Matière dentaire d'ubturation suivant la revendication 1, caractérisée en ce qu'elle contient 10 à 15% en poids, par rapport au mélange de substances de remplissage, de la substance de remplissage a).

3. Matière dentaire d'obturation suivant la revendication 1, caractérisée en ce qu'elle contient 25 à 35% en poids, par rapport au mélange de substances de remplissage, de la substance de remplissage b).

4. Matière dentaire d'obturation suivant la revendication 1, caractérisée en ce qu'elle contient 55 à 65% en poids, par rapport au mélange de substances de remplissage, d'une microsubstance de remplissage c).

5. Matière dentaire d'obturation suivant la revendication 1 et/ou 4, caractérisée en ce que la micro-substance de remplissage c) présente une dimension moyenne de particule de 30 à 60 nm.

6. Matière dentaire d'obturation suivant une ou plusieurs des revendications précédentes, caractérisée en ce que la substance de remplissage a) présente la répartition granulométrique suivante:

| | |
|---|---|
| 5% | 0,5– 1 µm |
| 10% | 1 – 2 µm |
| 20% | 2 – 5 µm |
| 20% | 5 –10 µm |
| 30% | 10 –20 µm |
| 15% | 20 –40 µm |

7. Matière dentaire d'obturation suivant une ou plusieurs des revendications précédentes, caractérisée en ce que la substance de remplissage b) présente la répartition granulométrique suivante:

| | |
|---|---|
| 5% | 0,2– 0,5 µm |
| 10% | 0,5– 1 µm |
| 15% | 1 – 2 µm |
| 45% | 2 – 5 µm |
| 20% | 5 –10 µm |
| 5% | 10 –15 µm |